# EUROPEAN PATENT SPECIFICATION

(11) **EP 3 474 727 B1**
(45) Date of publication and mention of the grant of the patent: **30.10.2019**
(21) Application number: 17731516.5
(22) Date of filing: 14.06.2017
(51) Int. Cl.: A61B 5/00, G06K 9/00

(54) **DEVICE AND METHOD FOR SKIN GLOSS DETECTION**
VORRICHTUNG UND VERFAHREN ZUR HAUTGLANZDETEKTION
DISPOSITIF ET PROCÉDÉ DE DÉTECTION DE BRILLANCE DE LA PEAU

(30) Priority: 27.06.2016 EP 16176320
(43) Date of publication of application: 01.05.2019
(73) Proprietor: Koninklijke Philips N.V., 5656 AE Eindhoven (NL)
(72) Inventor: VAN BREE, Karl Catharina, 5656 AE Eindhoven (NL); VELTHOVEN, Leo Jan, 5656 AE Eindhoven (NL)
(74) Representative: Kabuk, Yavuz
(86) International application number: PCT/EP2017/064483
(87) International publication number: WO 2018/001727

(56) References cited:
- US-A- 4 846 184
- US-A1- 2007 132 759
- US-A1- 2016 098 614

## Description

### FIELD OF THE INVENTION

The present invention relates to a device and a method for skin gloss detection.

### BACKGROUND OF THE INVENTION

Many men and women, particularly in Asia, are concerned about the shininess of their skin. They are seeking for efficient and simple solutions enabling measuring the amount of gloss (i.e. the gloss level of skin), which may be an interesting metric. Multiple on skin sensors are known for this purpose, many of which exploit measurement of the specular reflection component of one or multiple controlled light sources. But there is still a need for methods to measure apparent skin gloss more easily and efficiently.

US 4846184 A discloses a probe comprising a casing of which one face which will be in contact with the skin is provided with an aperture, is connected to a measuring device by means of a flexible connection in fiber optics comprising at least three optical conductors which, at a first end, are secured in the casing of the probe such as to face the aperture thereof, the first and second conductors having their first end portions directed respectively in a first and a second directions which are symmetrical to each other with respect to an axis extending normally through the aperture, while the third conductor has its first end portion directed in another direction than said second direction.

Further prior art can be found in US 2016/098614 A1 and US 2007/132759 A1.

### SUMMARY OF THE INVENTION

It is an object of the present invention to provide a device and method for skin gloss detection, which easily and efficiently allow determining the amount of skin gloss of a skin area.

In a first aspect of the present invention a device for skin gloss detection is presented, said device comprising:
- an illumination unit for illuminating a skin area at a flashing rate,
- an imaging unit for acquiring images of the skin area at an imaging rate, which is different than said flashing rate, wherein images of the skin area while the illumination by said illumination unit is on and images of the skin area while the illumination by said illumination unit is off are acquired, and
- a processing unit for processing acquired images by detecting the amount of gloss in the skin area from a specular reflection component and a diffuse component in at least one first partial image of the skin area representing a portion of an image acquired by the imaging unit while the skin area is illuminated by said illumination unit and at least one second partial image of the skin area representing a portion of an image acquired by the imaging unit while the skin area is not illuminated by said illumination unit.

In a further aspect of the present invention a method for skin gloss detection is presented, said method comprising:
- illuminating a skin area at a flashing rate,
- acquiring images of the skin area at an imaging rate which is different than said flashing rate, wherein images of the skin area while the illumination is on and images of the skin area while the illumination is off are acquired, and
- processing acquired images by detecting the amount of gloss in the skin area from a specular reflection component and a diffuse component in at least one first partial image of the skin area representing a portion of an image acquired while the skin area is illuminated and at least one second partial image of the skin area representing a portion of an image acquired while the skin area is not illuminated.

In yet further aspects of the present invention, there are provided a computer program which comprises program code means for causing a device as disclosed herein to perform the steps of the method disclosed herein when said computer program is carried out on the device as well as a non-transitory computer-readable recording medium that stores therein a computer program product, which, when executed by a device, causes the method disclosed herein to be performed.

Preferred embodiments of the invention are defined in the dependent claims. It shall be understood that the claimed method, computer program and medium have similar and/or identical preferred embodiments as the claimed device, in particular as defined in the dependent claims and as disclosed herein.

The present invention is based on the idea to make use of the rolling shutter effect. Shearing due to the rolling shutter appears to be a problem at first sight, but with the right processing it allows to actually capture as high as line rate. This means that the flashing frequency can be much above the human visibility of the flickering. The technology enabling measurement of skin gloss using the large installed base of user devices, such as smartphones or cameras, provides a great opportunity to go quickly to a large market. One problem with any camera based solution is the technology to accurately track facial features and face pose in video. According to the present invention, however, different embodiments of a solution are provided that overcomes said problem.

At least one partial image (e.g. a portion of a complete image) of the skin area while being illuminated and at least one partial image of the skin area while not being illuminated may be used for detecting the amount of gloss in the skin area. Partial hereby relates to the fact that when under-sampling temporally, the illumination can be 'still on' at the upper part of the image, and off at the bottom part of the image.

In an embodiment said imaging unit is configured to acquire images of the illuminated skin area at an imaging rate which is higher than said flashing rate. For instance, when recording images at an imaging rate of 120 images (also called image frames) per second and requiring alternately lit images, the illumination unit (also called flash) operates at half this frequency (60 fps) or lower, resulting in annoying but also potentially dangerous visibility of this flash modulation for epileptic sensitive people.

In an exemplary embodiment, the flashing rate is derived from a line rate and imaging rate of the imaging unit in such a way that in every other field (i.e. frame) the light is (substantially) alternating in each set of n lines. The image is generally read from the imaging unit line by line. The rate of this readout is the line rate. The flashing rate Ff may be chosen such that half the flashing cycle time Tf/2 = 1/(2^{∗}Ff) is an odd division factor of the frame capture time TN (where N indicate the number of lines in the frame including blanking lines). For simplicity, the flashing duty cycle may be chosen as (but is not limited to) 50%. This division factor then determines the value n, which is the number of lines with consecutive similar flash exposure (on or off). n will be a value between 1 and the number of images lines N. In practice, the flash (illumination unit) and the camera (imaging unit) are unsynchronized and the pattern will drift.

In another embodiment said illumination unit is configured to illuminate the skin area at a flashing rate which is a non-integer multiple higher than said imaging rate, in particular higher than two times said imaging rate. In this embodiment the knowledge about the rolling shutter effect is exploited to overcome this limit for the flashing rate by operating the imaging unit at a lower framerate and the flash at a non-integer factor higher framerate, to make sure that per image-line alternating flash and non-flash exposures can be recorded. This has the additional benefit of reducing computation time for face feature tracking as there are fewer image frames per second to process and register. A flashing rate between 1.0 (practically from 1.5) times the double imaging rate and half of the 'number of lines'-rate (= 1/Tns, where Tns is the time of n line exposures, wherein n is between 1 and the number of lines in a frame (including blanking lines)) may be applied.

The device may preferably further comprise a control unit for controlling the flashing rate and/or the imaging rate. The rates may be predetermined and fixed or may be controllable, e.g. by the user, for instance depending on the ambient light conditions, the skin type and/or color, etc. In another embodiment the control unit is configured to generate configurable temporal light patterns with modifiable duty cycles.

There are several options for processing of the acquired images to determine the skin gloss. In one embodiment said processing unit is configured to determine the specular reflection component in one or more sub-areas of said skin area and to determine the amount of skin gloss in said sub-areas by determining the ratio of specular reflection component in said one or more sub-areas to a diffuse component in said one or more sub-areas. The processing unit is particularly configured to determine skin sub-areas of the illuminated skin area which are substantially perpendicular to the optical axis of the imaging unit and to determine the amount of skin gloss from the determined skin sub-areas. The specular components are generally present at those perpendicular skin sub-areas, which may e.g. be determined by use of a face feature tracker in combination with a pose estimation algorithm. For instance, a decent map of these skin sub-areas may be determined. The values during illumination on are S+D. The values during illumination off are only D. Hereby, S is the specular component and D is the diffuse component). A first order approximation of the gloss for the perpendicular angle is S/D, which can be computed like ('S+D'-D)/D. Alternatively, also (S+D)/D is a ratio that provides a glossiness value, which after applied heuristics, compensating for distance based on the scale of the face features, can provide a robust estimation of first order skin glossiness.

According to another embodiment said processing unit may be configured to determine said skin sub-areas by use of a deformable model evaluating skin landmarks and/or pose estimation or by detecting flash components and diffuse components. As described above, the illumination adds a luminance component to the scene. It is one aspect of the present invention the rolling shutter effect is exploited. By summing up the image lines (in the skin area, e.g. the face area) the flash modulation can be detected in the image data. Since this is an un-synchronized system, this sets the expected modulation range. Under the assumption that skin objects, e.g. face objects, are larger than n number of lines, the modulation is typically very strong. The mere presence of strong modulation can be used to determine which areas to take into account when computing the amplitude of the modulation. In other words, 'If it is blinking it is probably the signal is the desired signal; if it is not blinking it can be ignored'.

The processing unit may further be configured to compensate one or more images of the skin area while not being illuminated by said illumination unit with a contribution of the illumination to a diffuse component estimation per color channel histogram shifting. When sub-tracking two images in order to find the components which are higher due to changing light conditions, 'skin structures' should first be accurately aligned. By taking the difference then, the light contribution (even in 3 color channels) is then obtained. Without this exact alignment, the edges of the skin patterns (freckles, moles, pores, ...) would possibly be measured instead. The skin is generally not flat though, which means more pixels in the skin area have to be registered to get a dense registration in order to warp the image properly for local alignment everywhere.

In another embodiment said processing unit is configured to determine a numerical skin gloss value per pixel, in particular by dividing a specular component value per pixel by a diffuse component value per pixel or by dividing the sum of a specular component value per pixel and a diffuse component value per pixel by the diffuse component value per pixel.

As mentioned above, said processing unit may be configured to determine a skin gloss map of the skin area indicating the amount of skin gloss per pixel or per group of pixels. This skin gloss map may then be outputted to the user, e.g. on a display, or an average skin gloss value may be determined for the skin areas for which the skin gloss map is determined.

The herein disclosed device may generally be any mobile user device having an illumination unit and an imaging unit. Exemplary (but non-limiting) embodiments include a smartphone, camera, laptop, or tablet.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a device according to the present invention,
Fig. 2 shows a diagram of the average luminance value per image frame F and two adjacent flash ON and flash OFF images,
Fig. 3 shows two example images with low gloss skin and high gloss skin,
Fig. 4 shows various examples of surface light reflection,
Fig. 5 shows face images to illustrate automatic face area tracking based on face landmark tracking,
Fig. 6 shows two example forehead regions with their color histograms,
Fig. 7 shows various difference images of the forehead region,
Fig. 8 shows an example binary mask indicating valid gloss values in the forehead region,
Fig. 9 shows an example output gloss map,
Fig. 10 shows three example frames while switching the flash at a flashing rate of 4.25 times the imaging rate, and
Fig. 11 shows a flow chart of a method for skin gloss detection according to the present invention.

### DETAILED DESCRIPTION OF THE INVENTION

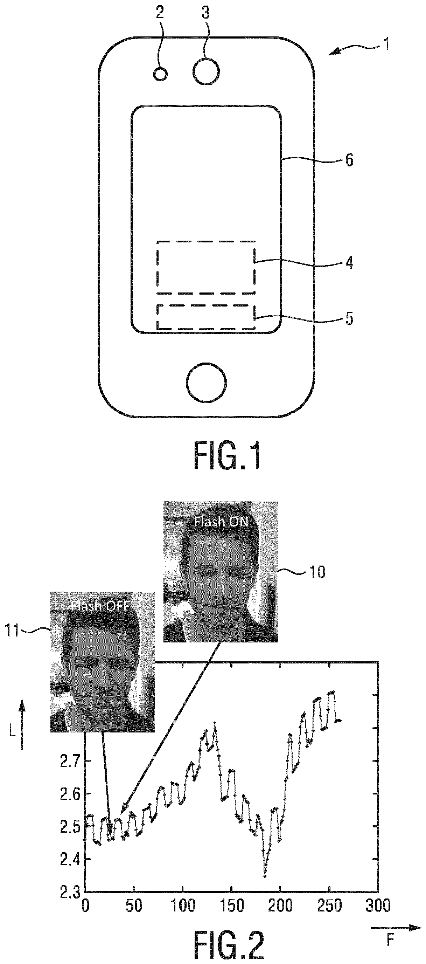

Fig. 1 shows a schematic diagram of an embodiment of a device 1 for skin gloss detection according to the present invention. The device may be a mobile user device, for instance a smartphone, camera, laptop, or tablet, which is available to many users for everyday use and which is adapted for the desired purpose of detecting skin gloss, e.g. by use of a software application ('app') that makes use of existing hardware components and evaluates data that are obtained by existing hardware components. Alternatively, the device may also be a dedicated device made particularly for the purpose of skin gloss detection (and optionally other purposes). In the example shown here and discussed in the following, the device 1 may be implemented as smartphone.

The device 1 comprises an illumination unit 2 for illuminating a skin area at a flashing rate. The illumination unit 2 may e.g. be the flash of a user device. In the following, the illumination unit is often simply referred to as flash, which shall, however, be generally understood as illumination unit.

The device 1 further comprises an imaging unit 3 for acquiring images of the skin area at an imaging rate which is different than said flashing rate. The imaging unit 3 may e.g. be the camera of a user device. In the following, the imaging unit is often simply referred to as camera, which shall, however, be generally understood as imaging unit.

The device 1 further comprises a processing unit 4 for processing acquired images and detecting the amount of gloss in the skin area from at least one partial (or complete) image of the skin area while being illuminated by said illumination unit 2 and at least one partial (or complete) image of the skin area while not being illuminated by said illumination unit 2. The processing unit 4 may e.g. be the processor of a user device. Generally, the processing unit 4 may also be an external processor, e.g. of an external computer, to which the acquired images are transmitted for processing to obtain information on the skin gloss. It is, however, preferred that the processing unit forms an integrated device with the illumination unit 2 and the imaging unit 3.

The device 1 may comprise further optional elements, such as a control unit 5 for controlling the illumination unit 2 and/or the imaging unit 3, in particular to control the flashing rate and/or the imaging rate. The task of the control unit 5 may, however, also be performed by the processor 4, or the flashing rate and/or the imaging rate may be predetermined and fixed so that an active control unit 5 may not be required.

The device 1 may further comprise a user interface 6, e.g. a display, keypad, touchscreen, etc., allowing the user to enter information, e.g. to start and stop skin gloss detection, change settings, enter personal information, etc., and enabling output of information, e.g. the detected skin gloss information or user instructions.

According to the present invention the illumination unit 2 is controlled in such a way that it is exactly known which image line is exposed with flash (i.e. illumination) on or off. Additionally, face feature tracking (or other means) may be used to accurately track the region, shape and pose of the face, in which the skin gloss of a skin area (e.g. of the forehead, the cheeks, the chin, etc.) shall be determined. Additionally, averaging (e.g. horizontal averaging) of the image content may be performed resulting in a strong 1D signal indicating the exact switching times per image line even more accurately.

In the following various embodiments will be explained in more detail. A first main embodiment extracts skin gloss values from alternating images. A second main embodiment creates and combines alternating illuminations per image line.

In the first main embodiment, for calculation of the amount of gloss (using a zero order approximation) the skin sub-areas, which are perpendicular to the imaging unit's optical axis (since at a smartphone the flash is mounted very close to the camera). These "perpendicular" sub-areas may e.g. be determined in one of the following two combined ways.

One way is model based. It may particularly use accurate face landmark detection and 3D face pose estimation. The fit of the 3D deformable model indicates the skin-sub-areas which are perpendicular substantially perpendicular to the imaging unit's optical axis. There are many different methods known for face landmark detection and 3D face pose estimation (e.g. as currently be found at http://blog.mashape.com/list-of-10-face-detection-recognition-apis/ or https://facedetection.com/software/). Further methods are listed by Tim Cootes at http://personalpages.manchester.ac.uk/staff/timothy.fcootes/tfc_publications.html. These include a computer algorithm to determine the visual location of the generic face features in the image and next fit a deformable 3D shape model to these generic face features in order to robustly estimate pose and surface angles. According to an embodiment spatial image locations are derived from the detected landmarks and the face pose, which are perpendicular to the camera. The 3D shape model registered to the generic face features provides an estimation of the skin normal vectors for each part of the skin.

According to another way the areas where the flash component is much larger than the diffuse component are used. The flash component is the illumination raise everywhere in the scene due to the flash (as later indicated by S+D). The specular component (S) of the flash illumination is much higher and only present at the location where the surface is close to perpendicular. On top of this, it is assumed that the ambient light contributes mostly to the baseline of D. The value of the diffuse component is either taken from the median of the delta in luminance in the face, since the majority of the face is not perpendicular to the optical axis of the camera. This works even more accurately by taking the areas from the above mentioned first way to determine these non-perpendicular areas.

Fig. 2 shows the average luminance value L (on the vertical axis) per image frame F (on the horizontal axis). Straight forward crossing detection with running means may be applied to find two adjacent flash ON and flash OFF images 10, 11.

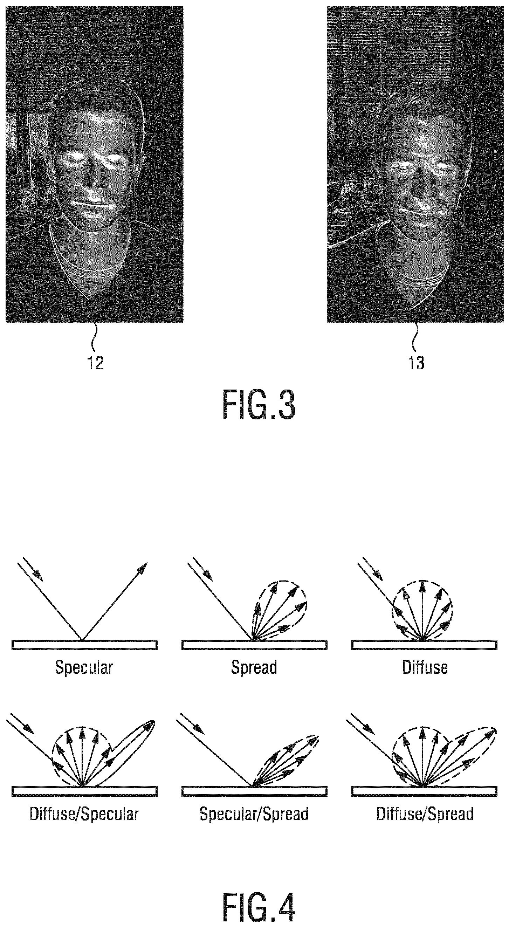

Fig. 3 shows two example images 12, 13 of a sequence of images with low gloss skin (image 12) and high gloss skin (image 13). Each image is the difference image of two adjacent flash ON and flash OFF images.

Fig. 4 shows various examples of surface light reflection.

In short, the ratio of the specular component and the diffuse component value may be used as an indicator for the amount of gloss.

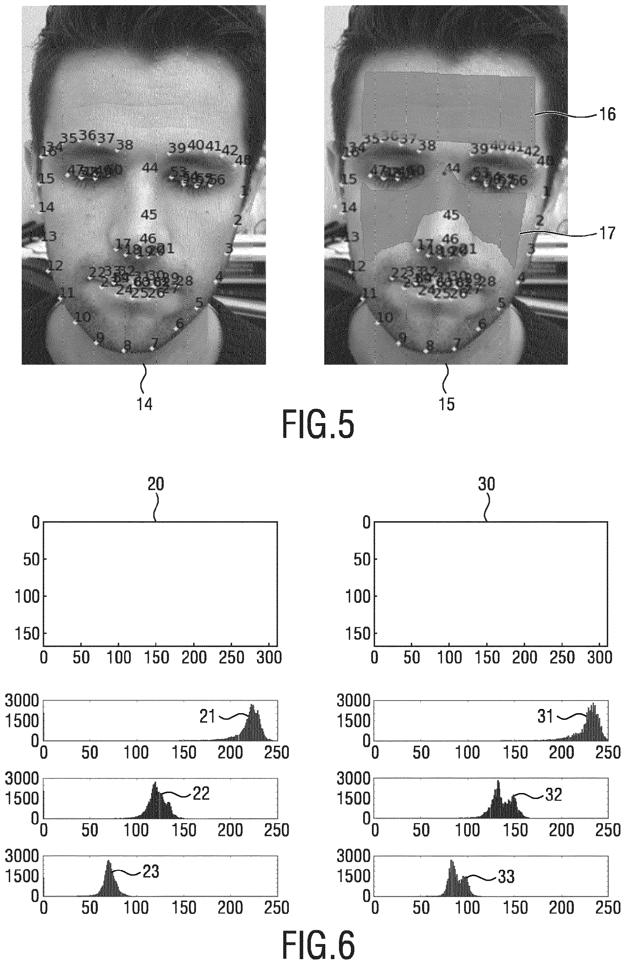

More refinement is achieved in another embodiment by compensating a non-flash image (e.g. image 11) with the contribution of the flash to the diffuse estimation per color channel histogram shifting. This histogram matching is preferably only applied in the tracked face area, derived from the face landmarks as shown in Fig. 5 depicting example images 14, 15 for illustrating automatic face area tracking based on face landmark tracking. In both images 14, 15 face landmarks are indicated by numbers. In image 15 the forehead region 16 and the cheek region 17 are indicated. Alternatively, the forehead area may include the area between the eyebrows (Tzone).

Fig. 6 shows two example forehead areas 20, 30 with their red 21, 31, green 22, 32 and blue 23, 33 color histograms. The forehead area 20 is obtained in an image without flash, the forehead area 30 is obtained in an image with flash. The histograms 31, 32, 33 are on average shifted up (right), plus an additional peak for the specular reflection components in the green and blue channels compared to the histograms 21, 22, 23. Histogram matching may thus be used to determine the flash contribution S+D more accurately per color channel. The flash does not simply contribute to the luminance equally across the light spectrum. In short, the superficial skin reflection is higher for green and blue. Red penetrates deeper into the skin.

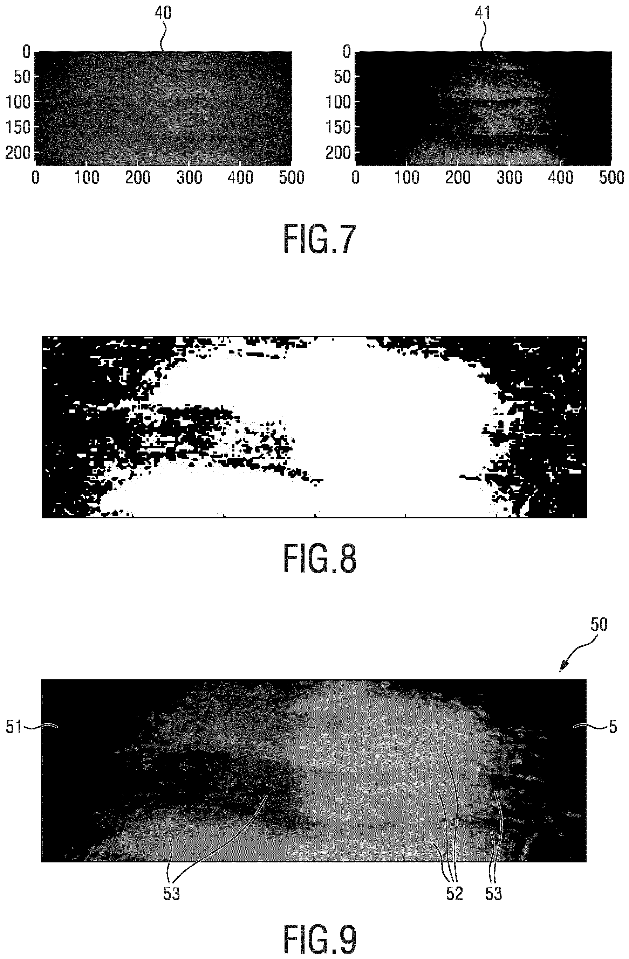

Fig. 7 shows various difference images 40, 41. Image 40 represents the difference between the registered flashed and non-flashed images. After first pore precise alignment and warping of the image patches. This holds the specular reflection component plus the diffuse reflection component. Image 41 represents the difference between 'the histogram matched non-flashed image' and the flashed image resulting only in the specular reflection component.

The face landmarks already give a robust registration/alignment of the face region per consecutive image frame. Additional accuracy may optionally be achieved by dense registration of image feature points inside this area.

The gloss estimation is preferably only computed for the skin sub-areas which are close to perpendicular to the camera axis and which show a significant specular component (e.g. having a value above a predetermined threshold). As mentioned, the 'perpendicularity' estimation for each pixel in the image can be coarsely determined by using robust 3D face model based face pose estimation. This combined sub-area can e.g. be illustrated by a binary mask as illustrated as an example in Fig. 8.

The numerical gloss value may be computed per pixel by dividing the specular component value by the diffuse component value and can be assigned to the skin area where the binary mask is valid. Alternatively, the ratio between the specular + diffuse component divided by the diffuse component may also be used as a coarse indicator for the amount of gloss.

In another embodiment, multiple camera view pairs from a video sequence (angled views on the face) are combined (e.g. by taking the maximum measured gloss) into a single gloss map, which is then assigned to the image with the 'most' frontal face present in the sequence of view pairs. This can yield a complete gloss map for each area of the face or the whole face. An example output gloss map 50 (with heat map color coding, i.e. areas 51 for low values, areas 52 for medium values and areas 53 for high values) for a forehead that was partly 'greased' with oil to illustrate the difference between low gloss (left part of the image) and medium/high gloss (right part of the image).

Further, in an embodiment the exposure time is automatically fixed by setting auto exposure during the first exposure while holding the flash ON and then locking the exposure. This results in a recording which is hardly ever clipped by over-exposure.

In the second main embodiment, the frame rate of the flash (i.e. the flashing rate) is a non-integer times higher than the frame rate of the camera (i.e. the imaging rate). This results in exposed images where the duty cycle of the flash is clearly visible in each single image. By deliberately choosing a flashing rate not starting in sync with each frame exposure, the phase of the illuminated image lines is shifting for every image frame. Although this scheme is more complex, it has the benefit of being able to run the flash at a modulation rate which is not perceivable by the human eye, which may be a requirement for the application of gloss measurement by a smartphone.

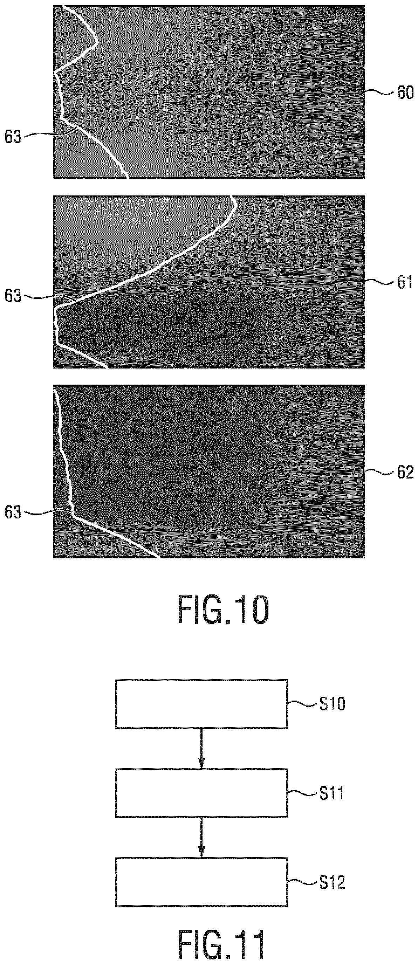

The timing of the flash could be exactly controlled in order to know which lines are exposed with flash on and which ones with flash off. For some existing base hardware (e.g. certain smartphones or cameras) the flash cannot be easily controlled in this much detail. When this is the case, as illustrated in this embodiment, the image line, at which the switching of the flash happens, can be extracted from the image robustly, e.g. by adding up the rows of the image and detecting the switching from this 1D modulation signal. Fig. 10 shows a few examples of this effect.

Fig. 10 particularly shows three example frames 60, 61, 62, which are acquired while switching the camera flash at 4.25 times the camera frame rate (i.e. the imaging rate), while recording a white piece of paper, in order to illustrate how each image line is differently illuminated. The overlaid line 63 indicates the average illumination value for each image line, indicating the duty cycle and phase of the flash.

The subsequent processing is rather similar to the processing explained above for the first main embodiment, while taking care of assigning each set of image lines to either flash ON or flash OFF state.

Fig. 11 shows a flow chart of a method for skin gloss detection according to the present invention. In a first step S10 a skin area is illuminates at a flashing rate. In a second step S11 images of the skin area are acquired at an imaging rate which is different than said flashing rate. In a third step acquired images processed and the amount of gloss in the skin area is detected from at least one partial (or complete) image of the skin area while being illuminated by said illumination unit and at least one partial (or complete) image of the skin area while not being illuminated by said illumination unit.

In summary, the present invention exploits the rolling shutter effect and adds some processing to measure skin gloss. Gloss can be detected at low flashing rates using complete flashed and non-flashed images. This can be achieved even when the image is teared by the flash going much faster with a flashing rate much higher than the imaging rate. With the proposed solution it is not a problem that the flash switches multiple times during the exposure of a frame.

In one embodiment a smartphone is set to record at a high frame rate (imaging rate) like e.g. 120 frames per second (fps), while (synchronously) switching the flashlight on and off at 60 fps. The recorded images now alternately consist of an ambient illuminated scene (consisting e.g. of a face) and a scene which is ambient illuminated plus the flash illumination. Using accurate face feature and pose tracking it has been shown to isolate and measure the specular reflection component with respect to the ambient component, which is a strong indication of the amount of glossiness of the skin. Additionally, in a second embodiment the camera runs a lower framerate while the flash operates at specific non-integer higher multiple of this frame rate, resulting, due to the rolling shutter effect, in different illuminations per set of image lines. Computer vision technology may be used to align the face areas despite the rolling shutter effect and measure different exposures for the same area resulting in similar gloss measurements.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for skin gloss detection, said device comprising:
- an illumination unit (2) configured to illuminate a skin area at a flashing rate,
- an imaging unit (3) configured to acquire images of the skin area at an imaging rate, which is different than said flashing rate, wherein images of the skin area while the illumination by said illumination unit is on and images of the skin area while the illumination by said illumination unit is off are acquired, and
- a processing unit (4) configured to process acquired images by detecting the amount of gloss in the skin area from a specular reflection component and a diffuse component in at least one first partial image of the skin area representing a portion of an image acquired by the imaging unit while the skin area is illuminated by said illumination unit and at least one second partial image of the skin area representing a portion of an image acquired by the imaging unit while the skin area is not illuminated by said illumination unit.

2. Device as claimed in claim 1,
wherein said imaging unit (3) is configured to acquire images of the illuminated skin area at an imaging rate which is higher than said flashing rate.

3. Device as claimed in claim 1,
wherein said illumination unit (2) is configured to illuminate the skin area at a flashing rate which is a multiple higher than said imaging rate.

4. Device as claimed in claim 3,
wherein said illumination unit (2) is configured to illuminate the skin area at a flashing rate, which is a non-integer multiple higher than two times said imaging rate.

5. Device as claimed in claim 1,
further comprising a control unit (5) for controlling the flashing rate and/or the imaging rate.

6. Device as claimed in claim 5,
wherein said control unit (5) is configured to generate configurable temporal light patterns with modifiable duty cycles.

7. Device as claimed in claim 1,
wherein said processing unit (4) is configured to determine the specular reflection component in one or more sub-areas of said skin area and to determine the amount of skin gloss in said sub-areas by determining the ratio of specular reflection component in said one or more sub-areas to a diffuse component in said one or more sub-areas.

8. Device as claimed in claim 1,
wherein said processing unit (4) is configured to determine skin sub-areas of the illuminated skin area which are substantially perpendicular to the optical axis of the imaging unit and to determine the amount of skin gloss from the determined skin sub-areas.

9. Device as claimed in claim 8,
wherein said processing unit (4) is configured to determine said skin sub-areas by use of a deformable model evaluating skin landmarks and/or pose estimation or by detecting flash components and diffuse components.

10. Device as claimed in claim 1,
wherein said processing unit (4) is configured to compensate one or more images of the skin area while not being illuminated by said illumination unit with a contribution of the illumination to a diffuse component estimation per color channel histogram shifting.

11. Device as claimed in claim 1,
wherein said processing unit (4) is configured to determine a numerical skin gloss value per pixel, in particular by dividing a specular component value per pixel by a diffuse component value per pixel or by dividing the sum of a specular component value per pixel and a diffuse component value per pixel by the diffuse component value per pixel.

12. Device as claimed in claim 1,
wherein said processing unit (4) is configured to determine a skin gloss map of the skin area indicating the amount of skin gloss per pixel or per group of pixels.

13. Device as claimed in claim 1,
wherein said device is a mobile user device, in particular a smartphone, camera, laptop, or tablet.

14. Method for skin gloss detection, said method comprising:
- illuminating, by means of an illumination unit (2), a skin area at a flashing rate,
- acquiring images, by means of an imaging unit (3), of the skin area at an imaging rate which is different than said flashing rate, wherein images of the skin area while the illumination is on and images of the skin area while the illumination is off are acquired, and
- processing, by means of a processing unit (4), acquired images by detecting the amount of gloss in the skin area from a specular reflection component and a diffuse component in at least one first partial image of the skin area representing a portion of an image acquired while the skin area is illuminated and at least one second partial image of the skin area representing a portion of an image acquired while the skin area is not illuminated.

15. Computer program comprising program code means for causing a device as claimed in claim 1 to carry out the steps of the method as claimed in claim 14 when said computer program is carried out on the device.

## Patentansprüche

1. Vorrichtung zur Hautglanzdetektion, wobei die Vorrichtung Folgendes umfasst:
- eine Beleuchtungseinheit (2), die konfiguriert ist, um eine Hautfläche mit einer Blitzgeschwindigkeit zu beleuchten,
- eine Bildgebungseinheit (3), die konfiguriert ist, um Bilder der Hautfläche mit einer Bildgebungsgeschwindigkeit zu erfassen, die von der Blitzgeschwindigkeit verschiedenen ist, wobei Bilder der Hautfläche, während die Beleuchtung durch die Beleuchtungseinheit an ist, und Bilder der Hautfläche, während die Beleuchtung durch die Beleuchtungseinheit aus ist, erfasst werden, und
- eine Verarbeitungseinheit (4), die konfiguriert ist, um erfasste Bilder durch Detektieren der Menge an Glanz in der Hautfläche aus einer spiegelnden Reflexionskomponente und einer diffusen Komponente in mindestens einem ersten Teilbild der Hautfläche, das einen Abschnitt eines Bildes darstellt, der von der Bildgebungseinheit erfasst wird, während die Hautfläche von der Beleuchtungseinheit beleuchtet wird, und mindestens einem zweiten Teilbild der Hautfläche, das einen Abschnitt eines Bildes darstellt, der von der Bildgebungseinheit erfasst wird, während die Hautfläche von der Beleuchtungseinheit nicht beleuchtet wird, zu verarbeiten.

2. Vorrichtung nach Anspruch 1,
wobei die Bildgebungseinheit (3) konfiguriert ist, um Bilder der beleuchteten Hautfläche mit einer Bildgebungsgeschwindigkeit zu erfassen, die höher ist als die Blitzgeschwindigkeit.

3. Vorrichtung nach Anspruch 1,
wobei die Beleuchtungseinheit (2) konfiguriert ist, um die Hautfläche mit einer Blitzgeschwindigkeit zu beleuchten, die um ein Mehrfaches höher ist als die Bildgebung sgeschwindigkeit.

4. Vorrichtung nach Anspruch 3,
wobei die Beleuchtungseinheit (2) konfiguriert ist, um die Hautfläche mit einer Blitzgeschwindigkeit zu beleuchten, die um ein nicht ganzzahliges Mehrfaches höher ist das Zweifache der Bildgebungsgeschwindigkeit.

5. Vorrichtung nach Anspruch 1,
weiter umfassend eine Steuerungseinheit (5) zum Steuern der Blitzgeschwindigkeit und/oder der Bildgebungsgeschwindigkeit.

6. Vorrichtung nach Anspruch 5,
wobei die Steuerungseinheit (5) konfiguriert ist, um konfigurierbare temporäre Lichtmuster mit modifizierbaren Einschaltdauern zu erzeugen.

7. Vorrichtung nach Anspruch 1,
wobei die Verarbeitungseinheit (4) konfiguriert ist, um die spiegelnde Reflexionskomponente in einer oder mehreren Subflächen der Hautfläche zu bestimmen und die Menge an Hautglanz in den Subflächen durch Bestimmen des Verhältnisses der spiegelnden Reflexionskomponente in der einen oder den mehreren Subflächen zu einer diffusen Komponente in der einen oder den mehreren Subflächen zu bestimmen.

8. Vorrichtung nach Anspruch 1,
wobei die Verarbeitungseinheit (4) konfiguriert ist, um Hautsubflächen der beleuchteten Hautfläche zu bestimmen, die im Wesentlichen senkrecht zu der optischen Achse der Bildgebungseinheit sind, und die Menge an Hautglanz von den bestimmten Hautsubflächen zu bestimmen.

9. Vorrichtung nach Anspruch 8,
wobei die Verarbeitungseinheit (4) konfiguriert ist, um die Hautsubflächen durch Verwendung eines verformbaren Modells, das Hautorientierungspunkte auswertet und/oder eine Schätzung vornimmt, oder durch Detektieren von Blitzkomponenten und diffusen Komponenten zu bestimmen.

10. Vorrichtung nach Anspruch 1,
wobei die Verarbeitungseinheit (4) konfiguriert ist, um ein oder mehrere Bilder der Hautfläche, während sie von der Beleuchtungseinheit nicht beleuchtet wird, durch einen Beitrag der Beleuchtung zu einer Schätzung der diffusen Komponente pro Farbkanalhistogrammverschiebung auszugleichen.

11. Vorrichtung nach Anspruch 1,
wobei die Verarbeitungseinheit (4) konfiguriert ist, um einen numerischen Hautglanzwert pro Pixel zu bestimmen, insbesondere durch Teilen eines Werts der spiegelnden Komponente pro Pixel durch einen Wert der diffusen Komponente pro Pixel oder durch Teilen der Summe eines Wertes der spiegelnden Komponente pro Pixel und eines Werts der diffusen Komponente pro Pixel durch den Wert der diffusen Komponente pro Pixel.

12. Vorrichtung nach Anspruch 1,
wobei die Verarbeitungseinheit (4) konfiguriert ist, um eine Hautglanzkarte der Hautfläche zu bestimmen, die die Menge an Hautglanz pro Pixel oder pro Pixelgruppe angibt.

13. Vorrichtung nach Anspruch 1,
wobei die Vorrichtung eine mobile Benutzervorrichtung ist, insbesondere ein Smartphone, eine Kamera, ein Laptop oder ein Tablet.

14. Verfahren zur Hautglanzdetektion, wobei das Verfahren Folgendes umfasst:
- Beleuchten einer Hautfläche mit einer Blitzgeschwindigkeit durch eine Beleuchtungseinheit (2),
- Erfassen von Bildern der Hautfläche mit einer Bildgebungsgeschwindigkeit, die von der Blitzgeschwindigkeit verschiedenen ist, durch eine Bildgebungseinheit (3), wobei Bilder der Hautfläche, während die Beleuchtung an ist, und Bilder der Hautfläche, während die Beleuchtung aus ist, erfasst werden, und
- Verarbeiten erfasster Bilder durch Detektieren der Menge an Glanz in der Hautfläche aus einer spiegelnden Reflexionskomponente und einer diffusen Komponente in mindestens einem ersten Teilbild der Hautfläche, das einen Abschnitt eines Bildes darstellt, der erfasst wird, während die Hautfläche beleuchtet wird, und mindestens einem zweiten Teilbild der Hautfläche, das einen Abschnitt eines Bildes darstellt, der erfasst wird, während die Hautfläche nicht beleuchtet wird, durch eine Verarbeitungseinheit (4).

15. Computerprogramm, umfassend Programmcodemittel, um zu bewirken, dass eine Vorrichtung nach Anspruch 1 die Schritte des Verfahrens nach Anspruch 14 ausführt, wenn das Computerprogramm auf der Vorrichtung ausgeführt wird.

## Revendications

1. Dispositif de détection de brillance de la peau, ledit dispositif comprenant :
- une unité d'éclairage (2) configurée pour éclairer une zone de la peau à un rythme de clignotement,
- une unité d'imagerie (3) configurée pour acquérir des images de la zone de la peau à un rythme d'imagerie qui est différent dudit rythme de clignotement, dans lequel les images de la zone de la peau tandis que l'éclairage par ladite unité d'éclairage est en marche et les images de la zone de la peau tandis que l'éclairage par ladite unité d'éclairage est arrêté sont acquises, et
- une unité de traitement (4) configurée pour traiter les images acquises en détectant la quantité de brillance dans la zone de la peau à partir d'une composante de réflexion spéculaire et d'une composante diffuse dans au moins une première image partielle de la zone de la peau représentant une partie d'une image acquise par l'unité d'imagerie tandis que la zone de la peau est éclairée par ladite unité d'éclairage et au moins une seconde image partielle de la zone de la peau représentant une partie d'une image acquise par l'unité d'imagerie tandis que la zone de la peau n'est pas éclairée par ladite unité d'éclairage.

2. Dispositif selon la revendication 1,
dans lequel ladite unité d'imagerie (3) est configurée pour acquérir des images de la zone de la peau éclairée à un rythme d'imagerie qui est supérieur audit rythme de clignotement.

3. Dispositif selon la revendication 1,
dans lequel ladite unité d'éclairage (2) est configurée pour éclairer la zone de la peau à un rythme de clignotement qui est un multiple supérieur audit rythme d'imagerie.

4. Dispositif selon la revendication 3,
dans lequel ladite unité d'éclairage (2) est configurée pour éclairer la zone de la peau à un rythme de clignotement qui est un multiple non entier supérieur à deux fois ledit rythme d'imagerie.

5. Dispositif selon la revendication 1,
comprenant en outre une unité de commande (5) pour commander le rythme de clignotement et/ou le rythme d'imagerie.

6. Dispositif selon la revendication 5,
dans lequel ladite unité de commande (5) est configurée pour générer des motifs lumineux temporels configurables avec des cycles de travail modifiables.

7. Dispositif selon la revendication 1,
dans lequel ladite unité de traitement (4) est configurée pour déterminer la composante de réflexion spéculaire dans une ou plusieurs sous-zones de ladite zone de la peau et déterminer la quantité de brillance de la peau dans lesdites sous-zones en déterminant le rapport de la composante de réflexion spéculaire dans une ou plusieurs sous-zones à une composante diffuse dans lesdites une ou plusieurs sous-zones.

8. Dispositif selon la revendication 1,
dans lequel ladite unité de traitement (4) est configurée pour déterminer des sous-zones de la zone de la peau éclairée qui sont sensiblement perpendiculaires à l'axe optique de l'unité d'imagerie et déterminer la brillance de la peau depuis les sous-zones de la peau déterminées.

9. Dispositif selon la revendication 8,
dans lequel ladite unité de traitement (4) est configurée pour déterminer lesdites sous-zones de la peau par utilisation d'un modèle déformable évaluant des repères de la peau et/ou une estimation de la pose ou par détection de composantes clignotantes et de composantes diffuses.

10. Dispositif selon la revendication 1,
dans lequel ladite unité de traitement (4) est configurée pour compenser une ou plusieurs images de la zone de la peau tandis qu'elles ne sont pas éclairées par ladite unité d'éclairage avec une contribution de l'éclairage à une estimation de la composante diffuse par décalage de l'histogramme de canaux de couleurs.

11. Dispositif selon la revendication 1,
dans lequel ladite unité de traitement (4) est configurée pour déterminer une valeur numérique de brillance de la peau par pixel, en particulier en divisant une valeur de composante spéculaire par pixel par une valeur de composante diffuse par pixel ou en divisant la somme d'une valeur de composante spéculaire par pixel et d'une valeur de composante diffuse par pixel par la valeur de composante diffuse par pixel.

12. Dispositif selon la revendication 1,
dans lequel ladite unité de traitement (4) est configurée pour déterminer une carte de brillance de la zone de la peau indiquant la quantité de brillance de la peau par pixel ou par groupe de pixels.

13. Dispositif selon la revendication 1,
dans lequel ledit dispositif est un dispositif utilisateur mobile, en particulier un smartphone, une caméra, un ordinateur de bureau ou une tablette.

14. Procédé de détection de brillance de la peau, ledit procédé comprenant :
- l'éclairage au moyen d'une unité d'éclairage (2) d'une zone de la peau à un rythme de clignotement,
- l'acquisition d'images au moyen de l'unité d'imagerie (3) de la zone de la peau à un rythme d'imagerie qui est différent dudit rythme de clignotement, dans lequel les images de la zone de la peau tandis que l'éclairage est en marche et les images de la zone de la peau tandis que l'éclairage est arrêté sont acquises et
- le traitement au moyen d'une unité de traitement (4) d'images acquises en détectant la quantité de brillance dans la zone de la peau à partir d'une composante de réflexion spéculaire et d'une composante diffuse dans au moins une première image partielle de la zone de la peau représentant une partie d'une image acquise tandis que la zone de la peau est éclairée et au moins une seconde image partielle de la zone de la peau représentant une partie d'une image acquise tandis que la zone de la peau n'est pas éclairée.

15. Programme d'ordinateur comprenant des moyens de codage de programme pour amener un dispositif selon la revendication 1 à effectuer les étapes du procédé selon la revendication 14 lorsque ledit programme d'ordinateur est effectué sur le dispositif.
